# EUROPÄISCHE PATENTSCHRIFT

(11) **EP 2 392 906 B1**
(45) Veröffentlichungstag und Bekanntmachung des Hinweises auf die Patenterteilung: **20.02.2019**
(21) Anmeldenummer: 11161101.8
(22) Anmeldetag: 05.04.2011
(51) Int. Cl.: G01M 99/00, G01N 21/93, G01N 21/90, A61L 2/28

(54) **Vorrichtung und Verfahren zum Überprüfen der Reinigungswirkung einer Reinigungsvorrichtung**
Method and device for checking the effectiveness of a cleaning device
Dispositif et procédé de vérification de l'effet nettoyant d'un dispositif de nettoyage

(30) Priorität: 01.06.2010 DE 102010029576
(43) Veröffentlichungstag der Anmeldung: 07.12.2011
(73) Patentinhaber: Krones AG, 93073 Neutraubling (DE)
(72) Erfinder: Wahl, Matthias, 93073 Neutraubling (DE); Winzinger, Frank, 93073 Neutraubling (DE)
(74) Vertreter: Grünecker Patent- und Rechtsanwälte PartG mbB

(56) Entgegenhaltungen:
- EP-A1- 0 764 479
- EP-A1- 1 281 446
- EP-A2- 0 672 900
- DE-A1- 3 324 449
- DE-A1- 4 101 731
- DE-A1- 4 437 103
- DE-A1- 10 065 941
- DE-U1- 29 803 507
- US-A1- 2006 261 156
- US-B1- 7 571 586

## Beschreibung

Die vorliegende Erfindung betrifft eine Vorrichtung und ein Verfahren zum Überprüfen der Reinigungswirkung einer Reinigungsvorrichtung, wobei die Reinigungsvorrichtung Teil einer Behälterbehandlungsanlage ist.

Ein Verfahren zur Reinigung von Gebinden, beispielsweise Fässern oder Kegs, wird in der EP 0 764 479 A1 beschrieben. Eine im Wesentlichen zeitgleich Überprüfung der Qualität des Reinigungsvorgangs wird dadurch ermöglicht, dass ein Indikator in das Gebinde eingebracht wird, und dass wenigstens die im letzten Teil der Nachspülphase ablaufende Nachspülflüssigkeit auf das Vorhandensein des Indikators überprüft wird.

Die EP 1 281 446 A1 offenbart eine Messvorrichtung zum Überwachen der Reinigung von Behältern in einer Reinigungsvorrichtung. Mittels eines Dummybehälters werden Substanzen im Reinigungswasser festgestellt.

Reinigungsvorrichtungen werden beispielsweise in Abfüllanlagen der Getränkeindustrie verwendet, um Behälter, wie etwa Flaschen, zu reinigen, insbesondere zu sterilisieren. Um die Reinigungswirkung einer Reinigungsvorrichtung zu überprüfen wird üblicherweise eine vorherbestimmte Verschmutzungsmenge manuell durch eine Bedienperson in einen Testbehälter eingebracht. Dieser Testbehälter wird dann, wiederum manuell, in die Reinigungsvorrichtung eingeschleust und nach der Reinigung im Labor auf den vorhandenen Restverschmutzungsgrad hin untersucht.

Ein Nachteil dieser bekannten Verfahren ist jedoch, dass es durch die Handhabung des Testbehälters durch die Bedienperson zu einer zusätzlichen Verschmutzung, insbesondere Verkeimung, des Testbehälters kommen kann, wodurch das Überprüfungsergebnis verfälscht und damit ungenau werden kann.

Daher ist es Aufgabe der vorliegenden Erfindung, eine Vorrichtung und ein Verfahren bereitzustellen, welche eine bessere Überprüfung der Reinigungswirkung einer Reinigungsvorrichtung erlauben.

Die Erfindung stellt eine Vorrichtung zum Überprüfen der Reinigungswirkung einer Reinigungsvorrichtung, wobei die Reinigungsvorrichtung Teil einer Behälterbehandlungsanlage ist, bereit, umfassend eine Einrichtung zum Einbringen und/oder Aufbringen einer vorherbestimmten Verschmutzungsmenge in und/oder auf einen Testbehälter, und eine Einrichtung zum Einschleusen des Testbehälters in die Behälterbehandlungsanlage.

Dadurch dass das Einbringen und/oder Aufbringen der vorherbestimmten Verschmutzungsmenge und das Einschleusen des Testbehälters in die Behälterbehandlungsanlage maschinell durchgeführt wird, kann eine Kontamination des Testbehälters durch eine Bedienperson verhindert oder wenigstens minimiert werden, und dadurch eine bessere Überprüfung der Reinigungswirkung der Reinigungsvorrichtung ermöglicht werden.

Die Einrichtung zum Einbringen und/oder Aufbringen einer vorherbestimmten Verschmutzungsmenge kann also derart ausgebildet sein, dass sie eine vorherbestimmte Anzahl oder Masse von Keimen und/oder eine vorherbestimmte Anzahl oder Masse von Schmutzpartikeln in und/oder auf dem Testbehälter anordnet.

Die vorherbestimmte Verschmutzungsmenge können beispielsweise 10, 100, 1000, 10.000, 100.000, 1 Million, 10 Millionen, 100 Millionen oder 1 Milliarde Keimen und/oder je nach Größe 10 bis 1 Million insbesondere 100 bis 1.000 Schmutzpartikeln entsprechen. Unter Verschmutzung kann man im weitesten Sinn auch Fette, Öle oder andere Flüssigkeiten oder Suspensionen verstehen, von denen beispielsweise 0,1 mg bis zu 100 mg eingebracht werden können.

Die Keime können einer Art von Keimen oder einer Mischung mehrerer Arten von Keimen entsprechen. Als Keime können hier Bakterien, Sporen von Bakterien und/oder Viren, insbesondere Krankheitserreger, bezeichnet werden. Insbesondere können Keime der Keimart "Bacillus Atrophaeus" und/oder Keime der Keimart "Bacillus Subtilis" verwendet werden.

Die vorherbestimmte Verschmutzungsmenge kann beispielsweise in einer Suspension vorliegen, wobei die Einrichtung zum Einbringen und/oder Aufbringen der vorherbestimmten Verschmutzungsmenge die Suspension in und/oder auf den Testbehälter einbringt und/oder aufbringt.

Als Reinigung kann hier die Entfernung einer Verschmutzung im Allgemeinen bezeichnet werden. Als Reinigung kann auch eine Sterilisierung bezeichnet werden. Als Reinigung kann auch die Entfernung von Verschmutzungen und die Sterilisation bezeichnet werden.

Die vorherbestimmte Verschmutzungsmenge kann Verschmutzungen, wie beispielsweise Staub und/oder Keime, wie beispielsweise Keime der Art "Bacillus Atrophaeus", enthalten.

Als Reinigungswirkung kann hier die erzielte Reinigung bezeichnet werden, also beispielsweise der Anteil der vorherbestimmten Schmutzmenge, der durch die Reinigung entfernt wurde. Im Falle einer Sterilisation kann als Reinigungswirkung auch die erreichte Sterilität bezeichnet werden, also beispielsweise der Anteil der vorherbestimmten Keimanzahl, der durch die Reinigung entfernt und/oder abgetötet wurde.

Die Reinigungsvorrichtung kann mehrere Reinigungselemente umfassen. Dadurch können Behälter an unterschiedlichen Positionen in der Behälterbehandlungsanlage gereinigt werden.

Die Reinigungsvorrichtung kann einen Rinser und/oder eine Sterilisationseinrichtung umfassen. Die Reinigungsvorrichtung kann auch einem Rinser und/oder einer Sterilisationseinrichtung entsprechen. Im Fall einer Sterilisationseinrichtung kann die Sterilisationswirkung der Sterilisationseinrichtung überprüft werden.

Eine Sterilisationseinrichtung kann beispielsweise Wasserstoffperoxid (H₂O₂) oder Peressigsäure oder Ozon oder Dampf oder Schaum als Sterilisationsmittel verwenden. Alternativ oder zusätzlich kann die Sterilisationseinrichtung eine oder mehrere Strahlungsquellen für ultraviolette Strahlung und/oder Wärmestrahlung und/oder Mikrowellenstrahlung und/oder andere Strahlungen enthalten.

Im weitesten Sinne kann die Reinigungsvorrichtung auch eine Heizvorrichtung, beispielsweise eine Heizvorrichtung einer Blasmaschine, umfassen oder einer solchen Heizvorrichtung entsprechen. Durch die Erwärmung der Behälter in der Heizvorrichtung kann es nämlich zu einer wenigstens teilweisen Sterilisation des Testbehälters kommen. Ebenso kann die Reinigungsvorrichtung eine oder mehrere UV-Lampen und/oder eine oder mehrere Mikrowellenerzeugungsvorrichtungen und/oder eine Dampferzeugungseinrichtung und/oder eine Plasmaerzeugungseinrichtung und/oder eine Elektronenstrahlerzeugungseinrichtung (E-beam) und/oder eine Ozonerzeugungseinrichtung umfasen. Denkbar wäre hier auch eine Röntgen- und/oder Gammastrahlungserzeugungseinrichtung

Bei dem Testbehälter kann es sich um einen im Betrieb der Behälterbehandlungsanlage verwendbaren Behälter, insbesondere einen Vorformling einer Blasmaschine oder eine Flasche, handeln. Dadurch kann das Verfahren mit geringem Aufwand durchgeführt werden.

Die Einrichtung zum Einbringen und/oder Aufbringen der vorherbestimmten Verschmutzungsmenge kann derart ausgebildet sein, dass die vorherbestimmte Verschmutzungsmenge auf einem vorherbestimmten oder gewünschten Oberflächenbereich in und/oder auf dem Testbehälter angeordnet wird. Dadurch kann die Reinigungswirkung für bestimmte, beispielsweise schwer zugängliche, Oberflächenbereiche der Behälter überprüft werden. Der vorherbestimmte oder gewünschte Oberflächenbereich kann einen oder mehrere, zusammenhängende, teilweise zusammenhängende oder nicht zusammenhängende, Teilbereiche umfassen.

Durch das maschinelle Einbringen und/oder Aufbringen der vorherbestimmten Verschmutzungsmenge kann es auch möglich sein, eine präzisere Anordnung und/oder eine präzisere Dosierung der vorherbestimmten Verschmutzungsmenge zu erreichen als durch ein manuelles Anordnen durch eine Bedienperson.

Die Einrichtung zum Einbringen und/oder Aufbringen der vorherbestimmten Verschmutzungsmenge kann insbesondere mehrere Elemente zum Einbringen und/oder Aufbringen der vorherbestimmten Verschmutzungsmenge umfassen. Beispielsweise können für unterschiedliche Teilbereiche in und/oder auf dem Testbehälter unterschiedliche Elemente zum Einbringen und/oder Aufbringen der vorherbestimmten Verschmutzungsmenge vorgesehen sein. Dadurch kann auf einfache Weise die vorherbestimmte Verschmutzungsmenge in mehreren Teilbereichen angeordnet werden.

Beispielsweise kann die Einrichtung zum Einbringen und/oder Aufbringen der vorherbestimmten Verschmutzungsmenge ein erstes Element, das einen Teil der vorherbestimmten Verschmutzungsmenge auf einer äußeren Oberfläche des Testbehälters aufbringt und ein zweites Element, das einen Teil der vorherbestimmten Verschmutzungsmenge in den Testbehälter einbringt, insbesondere auf einer inneren Oberfläche des Testbehälters aufbringt, umfassen.

Die Einrichtung zum Einbringen und/oder Aufbringen der vorherbestimmten Verschmutzungsmenge und/oder die Einrichtung zum Einschleusen des Testbehälters in die Behälterbehandlungsanlage können derart angeordnet und/oder ausgebildet sein, dass das Einschleusen des Testbehälters vor, nach und/oder während des Einbringens und/oder Aufbringens der vorherbestimmten Verschmutzungsmenge durchgeführt wird.

Beispielsweise kann die Einrichtung zum Einbringen und/oder Aufbringen der vorherbestimmten Verschmutzungsmenge im Bereich einer Transportvorrichtung der Behälterbehandlungsanlage und/oder im Bereich der Einrichtung zum Einschleusen des Testbehälters in die Behälterbehandlungsanlage angeordnet sein.

Die Vorrichtung zum Überprüfen der Reinigungswirkung kann außerdem eine Einrichtung zum Ausschleusen des Testbehälters aus der Behälterbehandlungsanlage umfassen. Damit kann der Testbehälter auch im Betrieb der Behälterbehandlungsanlage an einer gewünschten Stelle der Behälterbehandlungsanlage aus der Behälterbehandlungsanlage ausgeschleust werden.

Die Einrichtung zum Einschleusen des Testbehälters und/oder die Einrichtung zum Ausschleusen des Testbehälters können jeweils mehrere Elemente zum Einschleusen beziehungsweise Ausschleusen des Testbehälters umfassen, welche an unterschiedlichen Stellen der Behälterbehandlungsanlage angeordnet werden können. Mit anderen Worten können die Elemente zum Einschleusen des Testbehälters und/oder die Elemente zum Ausschleusen des Testbehälters variabel positionierbar sein. Dadurch können unterschiedliche Abschnitte der Behälterbehandlungsanlage durch den Testbehälter durchlaufen werden, wodurch wiederum die Reinigungswirkung von Reinigungselementen in einzelnen Abschnitten der Behälterbehandlungsanlage überprüft werden kann.

Die Behälterbehandlungsanlage kann einer Abfüllanlage, insbesondere einer Abfüllanlage der Getränkeindustrie, entsprechen.

Die Behälterbehandlungsanlage kann insbesondere eine Blasmaschine zum Herstellen von Kunststoffbehältern aus Vorformlingen, auch Preforms genannt, umfassen oder einer solchen entsprechen. Die Blasmaschine kann eine Preformzuführung, eine Heizvorrichtung zum Erwärmen der Preforms und/oder ein Blasmodul zum Blasen, insbesondere Streckblasen, der Kunststoffbehälter in Blasformen umfassen.

Die Behälterbehandlungsanlage kann außerdem einen Füller zum Befüllen von Behältern, insbesondere von in der Blasmaschine hergestellten Kunststoffbehältern, umfassen. Die Behälterbehandlungsanlage kann außerdem eine Spritzmaschine für die Herstellung von Vorformlingen, eine Etikettiervorrichtung, wenigstens eine Transportvorrichtung und/oder eine Regeneriervorrichtung zum Regenerieren von Mehrwegkunststoffflaschen umfassen.

Die Vorrichtung zum Überprüfen der Reinigungswirkung kann auch mehrere Einrichtungen zum Einbringen und/oder Aufbringen einer vorherbestimmten Verschmutzungsmenge umfassen. Diese können insbesondere an unterschiedlichen Positionen, insbesondere innerhalb einer Behälterbehandlungsanlage, angeordnet werden.

Eine oder mehrere Einrichtungen zum Einbringen und/oder Aufbringen einer vorherbestimmten Verschmutzungsmenge können auch zur Bedienung bzw. Überprüfung von jeweils mehreren Behälterbehandlungsanlagen vorgesehen sein.

Ein Reinigungselement der Behälterbehandlungsanlage kann zwischen einer Heizvorrichtung einer Blasmaschine und einem Blasmodul der Blasmaschine angeordnet sein. Ein weiteres Reinigungselement kann beispielsweise nach dem Blasmodul und vor einem Füller angeordnet sein. Alternativ oder zusätzlich kann ein Reinigungselement vor einer Regeneriervorrichtung zum Regenerieren von Mehrwegkunststoffflaschen vorgesehen sein.

Die Einrichtung zum Einschleusen des Testbehälters kann derart angeordnet und/oder ausgebildet sein, dass der Testbehälter vor, insbesondere unmittelbar vor, der Reinigungsvorrichtung, insbesondere vor einem vorherbestimmten Reinigungselement der Reinigungsvorrichtung, eingeschleust oder eingebracht wird.

Die Einrichtung zum Einschleusen des Testbehälters kann insbesondere derart angeordnet und/oder ausgebildet sein, dass der Testbehälter vor, insbesondere unmittelbar vor, einer Preformzuführung, einer Heizvorrichtung einer Blasmaschine, einer Etikettiervorrichtung, einem Rinser, einer Sterilisationseinrichtung, einer Behälterbeschichtungsstation, einem Füller und/oder einem Verschließer eingeschleust wird. Die Einrichtung zum Einschleusen des Testbehälters kann auch derart angeordnet und/oder ausgebildet sein, dass der Testbehälter nach, insbesondere unmittelbar nach, einem Füller, einem Rinser, einer Sterilisationseinrichtung, einer Behälterbeschichtungsstation, einer Etikettiervorrichtung, einem Blasmodul oder in einer Preformzuführung oder zwischen einer Heizvorrichtung einer Blasmaschine und einem Blasmodul, eingeschleust wird.

Die Einrichtung zum Ausschleusen des Testbehälters kann derart angeordnet und/oder ausgebildet sein, dass der Testbehälter nach, insbesondere unmittelbar nach, der Reinigungsvorrichtung, insbesondere nach einem vorherbestimmten Reinigungselement der Reinigungsvorrichtung, ausgeschleust oder ausgebracht wird.

Die Einrichtung zum Ausschleusen des Testbehälters kann insbesondere derart angeordnet und/oder ausgebildet sein, dass der Testbehälter vor, insbesondere unmittelbar vor, einer Heizvorrichtung einer Blasmaschine, einem Füller oder einem Verschließer ausgeschleust wird. Die Einrichtung zum Ausschleusen des Testbehälters kann auch derart angeordnet und/oder ausgebildet sein, dass der Testbehälter nach, insbesondere unmittelbar nach, einem Verschließer, einem Rinser, einer Sterilisationseinrichtung, einer Behälterbeschichtungsstation, einer Etikettiervorrichtung, einem Blasmodul und/oder einem Füller oder in einer Preformzuführung oder zwischen einer Heizvorrichtung einer Blasmaschine und einem Blasmodul ausgeschleust wird.

Wenn die Behälterbehandlungsanlage eine Packvorrichtung umfasst kann der Testbehälter vor, insbesondere unmittelbar vor, der Packvorrichtung eingeschleust und nach, insbesondere unmittelbar nach, der Packvorrichtung ausgeschleust werden. Wenn in der Packvorrichtung nur verschlossene Behälter in Gebinde gepackt werden sollen, kann in diesem Fall die vorherbestimmte Verschmutzungsmenge auch nur auf der Außenseite des Testbehälters, insbesondere eines Verschlusses des Testbehälters und/oder eines Etiketts des Testbehälters, aufgebracht werden.

Die Behälterbehandlungsanlage kann auch eine Blasmaschine umfassen, die ein Inmold-Labellingelement umfasst. Beim Inmold-Labelling werden Etiketten in die Blasformen des Blasmoduls eingebracht. In diesem Fall kann das Aufbringen der vorherbestimmten Verschmutzungsmenge auf den Testbehälter ein Aufbringen der vorherbestimmten Verschmutzungsmenge auf ein Etikett vor dem Einbringen des Etiketts in eine Blasform umfassen.

Wenn die Anlage eine Spritzmaschine zur Herstellung von Vorformlingen umfasst, kann das Einschleusen des Testbehälters nach, insbesondere unmittelbar nach, der Spritzmaschine erfolgen.

Die Vorrichtung zum Überprüfen der Reinigungswirkung umfasst außerdem eine Einrichtung zum Bestimmen einer Restverschmutzungsmenge nach der Reinigung des Testbehälters mit der Reinigungsvorrichtung.

Die Einrichtung zum Bestimmen einer Restverschmutzungsmenge kann beispielsweise einen optischen Sensor und/oder einen Detektor für biologische und/oder chemische Verunreinigungen, wie zum Beispiel einen Schnüffler, umfassen. Dadurch kann eine weitere direkte Interaktion des Testbehälters mit einer Bedienperson zum Bestimmen der Restverschmutzungsmenge vermieden werden, wodurch eine weitere mögliche Kontamination oder Verschmutzung des Testbehälters vermieden oder wenigstens reduziert werden kann.

Die Einrichtung zum Ausschleusen des Testbehälters und/oder die Einrichtung zum Bestimmen einer Restverschmutzungsmenge können derart angeordnet und/oder ausgebildet sein, dass das Bestimmen der Restverschmutzungsmenge vor, nach und/oder während des Ausschleusens des Testbehälters aus der Behälterbehandlungsanlage durchgeführt wird. Die Einrichtung zum Ausschleusen des Testbehälters und/oder die Einrichtung zum Bestimmen einer Restverschmutzungsmenge können zudem einen Puffer für einen oder mehrere Testbehälter umfassen, in dem eine Nährlösung in den einen oder in die mehreren Testbehälter eingebracht wird, um nach einem bestimmten Zeitraum eine Keimvermehrung beobachten oder messen zu können. Insbesondere kann die Vermehrung ebenfalls über die Einrichtung zum Bestimmen der Restverschmutzungsmenge, insbesondere automatisch, beobachtet oder gemessen werden.

Die Vorrichtung zum Überprüfen der Reinigungswirkung umfasst außerdem ein Steuerungselement, das derart konfiguriert ist, dass wenigstens ein Betriebsparameter der Behälterbehandlungsanlage, insbesondere der Reinigungsvorrichtung, in Abhängigkeit von der bestimmten Restverschmutzungsmenge geregelt wird. Dadurch kann die Effizienz der Reinigungsmaschine verbessert werden.

Das Steuerungselement kann derart konfiguriert sein, dass es einen Vergleich der bestimmten Restverschmutzungsmenge mit einem vorherbestimmten Wert, insbesondere mit der vorherbestimmten Verschmutzungsmenge, durchführt. Dadurch kann die Reinigungswirkung der Reinigungsvorrichtung quantifiziert werden.

Das Steuerungselement kann derart ausgebildet sein, dass es, wenn der Vergleich der bestimmten Restverschmutzungsmenge mit dem vorherbestimmten Wert ein vorherbestimmtes Kriterium nicht erfüllt, eine Ausgabe an eine Bedienperson ausgibt, beispielsweise in Form eines optisches und/oder akustisches Warnsignals. Dadurch kann beispielsweise eine Bedienperson benachrichtigt werden, wenn die Restverschmutzung einen zu hohen Wert annimmt.

Wenn beispielsweise bestimmt wird, dass die bestimmte Restverschmutzungsmenge größer als ein vorherbestimmter Schwellenwert ist, kann die Reinigungswirkung der Reinigungsvorrichtung erhöht werden, indem wenigstens ein Betriebsparameter der Reinigungsvorrichtung und/oder der Behälterbehandlungsanlage angepasst wird. Wenn dagegen bestimmt wird, dass die bestimmte Restverschmutzungsmenge kleiner als ein vorherbestimmter Schwellenwert ist, kann die Reinigungswirkung der Reinigungsvorrichtung auch erniedrigt werden, wodurch beispielsweise der Verbrauch an Reinigungsmitteln in der Reinigungsvorrichtung reduziert werden kann. Hierzu kann sowohl die Einrichtung zum Bestimmen einer Restverschmutzungsmenge als auch die Einrichtung zum Einschleusen oder Ausschleusen des Testbehälters oder die Einrichtung zum Einbringen und/oder Aufbringen einer vorherbestimmten Verschmutzungsmenge ein weiteres Steuerungselement umfassen, welches mit dem oben genannten Steuerungselement verbunden ist.

Der wenigstens eine Betriebsparameter kann einer Konzentration einer Sterilisationsflüssigkeit, einer Menge einer Sterilisationsflüssigkeit, einer Temperatur einer Sterilisationsflüssigkeit, einem Einspritzdruck einer Sterilisationsflüssigkeit, einer Position am Testbehälter für die Aufbringung der vorherbestimmten Verschmutzungsmenge, beispielsweise über eine Eintauchtiefe einer Lanze, einer Strahlungsstärke einer UV-Lampe und/oder einer Mikrowelleneinrichtung, einer Art Sterilisationsmedium für die Behälter, beispielsweise Wasserstoffperoxid, Peressigsäure oder Seifenlauge, einer verwendeten Sterilisationseinrichtung, einer Reinigungszeit für die Vorrichtungen und/oder die Behälter, einem Blasdruck einer Sterilisationsflüssigkeit und/oder einem Umgebungsdruck in der Behälterbehandlungsanlage oder in einem Teil der Behälterbehandlungsanlage entsprechen. Durch den Umgebungsdruck in der Behälterbehandlungsanlage oder in einem Teil der Behälterbehandlungsanlage kann die Sterilität der Atmosphäre in der Behälterbehandlungsanlage oder in dem Teil der Behälterbehandlungsanlage beeinflusst werden. Ein Überdruck in der Behälterbehandlungsanlage oder in dem Teil der Behälterbehandlungsanlage kann beispielsweise ein Eindringen von Verschmutzungen verhindern oder wenigstens erschweren.

Die Einrichtung zum Einbringen und/oder Aufbringen der vorherbestimmten Verschmutzungsmenge kann ein Trocknungselement zum Trocknen des Testbehälters umfassen. Dadurch kann beispielsweise der Testbehälter getrocknet werden, nachdem die vorherbestimmte Verschmutzungsmenge in Form einer Suspension in den Testbehälter eingebracht und/oder auf den Testbehälter aufgebracht wurde. Das Trocknungselement kann auch einer Heizvorrichtung einer Blasmaschine entsprechen.

Die Einrichtung zum Einbringen und/oder Aufbringen der vorherbestimmten Verschmutzungsmenge kann in einer Zuführung eine oder mehrere Kapillaren umfassen. Dieses Prinzip ist beispielsweise für eine andere Anwendung aus der DE 10 2008 037160 A1 bekannt und dient zu einer sehr genauen Dosierung.

Alternativ oder zusätzlich kann die Einrichtung zum Einbringen und/oder Aufbringen der vorherbestimmten Verschmutzungsmenge einen Durchflussmesser umfassen. Dadurch kann eine genaue Dosierung der vorherbestimmten Verschmutzungsmenge erreicht werden. Insbesondere kann der Durchflussmesser ein induktiv messender Durchflussmesser sein.

Alternativ oder zusätzlich kann die Einrichtung zum Einbringen und/oder Aufbringen der vorherbestimmten Verschmutzungsmenge eine Wägezelle, insbesondere zur Bestimmung der vorherbestimmten Verschmutzungsmenge, umfassen. Auf diese Weise kann das eingebrachte Gewicht einer Verschmutzungsmenge in den Testbehälter gemessen werden und nach einem Erreichen eines vorherbestimmten Sollwerts die Einbringung über eine Regelung gestoppt werden.

Alternativ oder zusätzlich kann die Einrichtung zum Einbringen und/oder Aufbringen der vorherbestimmten Verschmutzungsmenge einen optischen Sensor, wie beispielsweise eine Kamera, umfassen, insbesondere wobei die eingebrachte Verschmutzungsmenge über den optischen Sensor, beispielsweise die Kamera, bestimmt wird und nach einem Erreichen eines vorherbestimmten Sollwerts die Einbringung über eine Regelung gestoppt wird. Hierzu kann die Verschmutzung eingefärbt werden. Die eingebrachte Verschmutzungsmenge kann beispielsweise gemäß einem Verfahren nach der CN1654962 (A) bestimmt werden, bei dem eine CCD-Hochgeschwindigkeitskamera verwendet wird.

Zur Bestimmung der eingebrachten Verschmutzungsmenge können alternativ oder zusätzlich ein oder mehrere Piezodosierventile verwendet werden.

Die Einrichtung zum Einbringen und/oder Aufbringen der vorherbestimmten Verschmutzungsmenge kann wenigstens eine Düse umfassen. Die wenigstens eine Düse kann über einer Transportvorrichtung zum Transportieren des Testbehälters angeordnet sein.

Alternativ oder zusätzlich kann die Einrichtung zum Einbringen und/oder Aufbringen der vorherbestimmten Verschmutzungsmenge wenigstens ein Kontaktelement umfassen, wobei das wenigstens eine Kontaktelement und/oder die Einrichtung zum Einbringen und/oder Aufbringen der vorherbestimmten Verschmutzungsmenge derart ausgebildet sind, dass das wenigstens eine Kontaktelement mit einer Oberfläche des Testbehälters in direkten Kontakt gebracht werden kann und dabei, wenigstens teilweise, die vorherbestimmte Verschmutzungsmenge auf die Oberfläche aufbringt. Das wenigstens eine Kontaktelement kann insbesondere ein poröses Material wie Schaumstoff umfassen. Dadurch kann die vorherbestimmte Verschmutzungsmenge, wenn diese in einer Suspension bereitgestellt wird, vom wenigstens einen Kontaktelement aufgenommen werden. Das wenigstens eine Kontaktelement kann als Rolle ausgebildet sein, die auf einer Oberfläche des Testbehälters abrollbar ist.

Die Einrichtung zum Einbringen und/oder Aufbringen der vorherbestimmten Verschmutzungsmenge kann als Rundläufer oder linear ausgebildet sein.

Die Einrichtung zum Einbringen und/oder Aufbringen der vorherbestimmten Verschmutzungsmenge und/oder die Einrichtung zum Einschleusen des Testbehälters können jeweils ein oder mehrere Behandlungselemente der Behälterbehandlungsanlage umfassen oder solchen entsprechen. Durch die Nutzung eines Behandlungselements der Behälterbehandlungsanlage können Materialkosten eingespart werden.

Die Einrichtung zum Einbringen und/oder Aufbringen der vorherbestimmten Verschmutzungsmenge kann beispielsweise einem Preformrinser entsprechen oder einen solchen umfassen. Dazu kann der Preformrinser, insbesondere temporär, derart ausgebildet sein, dass er, beispielsweise anstelle von ionisierter Luft, die vorherbestimmte Verschmutzungsmenge, beispielsweise in Form einer Suspension, abgibt.

Die Einrichtung zum Einbringen und/oder Aufbringen der vorherbestimmten Verschmutzungsmenge kann derart ausgebildet sein, dass der Testbehälter an der Außenfläche des Rumpfes gegriffen oder gehaltert wird, insbesondere derart, dass vorherbestimmte oder gewünschte Flächen des Testbehälters freiliegen. Auf diese Weise kann eine Anordnung der vorherbestimmten Verschmutzungsmenge auf möglichst allen kritischen Flächen des Testbehälters, beispielsweise der Außenfläche des Mundstücks und/oder auf der kompletten Innenseite, gewährleistet werden.

Die Einrichtung zum Einbringen und/oder Aufbringen der vorherbestimmten Verschmutzungsmenge kann auch einen Heizdorn und/oder eine Reckstange einer Blasmaschine der Behälterbehandlungsanlage umfassen.

Die Einrichtung zum Einschleusen des Testbehälters kann beispielsweise eine Preformzuführung umfassen oder einer solchen entsprechen.

Die Vorrichtung zum Überprüfen der Reinigungswirkung kann außerdem ein Element zum Reinigen, insbesondere Sterilisieren, des Testbehälters vor dem des Einbringen und/oder Aufbringen der vorherbestimmten Verschmutzungsmenge umfassen. Dadurch kann eine noch bessere Überprüfung der Reinigungswirkung der Reinigungsvorrichtung ermöglicht werden, da eine eventuell vorhandene Verschmutzung des Testbehälters vor dem des Einbringen und/oder Aufbringen der vorherbestimmten Verschmutzungsmenge entfernt oder wenigstens minimiert wird.

Die Vorrichtung zum Überprüfen der Reinigungswirkung kann außerdem den Testbehälter umfassen, insbesondere wobei der Testbehälter ein Messdummy ist.

Als Messdummy ist hier ein Element zu verstehen, welches die Form eines Vorformlings oder eines Behälters aufweist. Ein Messdummy kann auch dieselben Abmessungen wie ein Vorformling oder ein Behälter aufweisen. Insbesondere kann ein Messdummy auch dasselbe Material wie ein Vorformling oder ein Behälter umfassen.

Ein Messdummy kann auch aufblasbar sein. Mit anderen Worten kann der Messdummy in einer Blasform einer Blasmaschine zu einem Kunststoffbehälter ausformbar sein. Alternativ kann der Messdummy derart ausgebildet sein, dass er in einer Blasform einer Blasmaschine nicht zu einem Kunststoffbehälter ausformbar ist, insbesondere also seine Form nicht oder nur geringfügig ändert.

Im und/oder am Messdummy kann wenigstens ein Messelement zum Bestimmen wenigstens eines Parameterwertes für den Betrieb einer Blasmaschine, einer Behälterbehandlungsanlage, einer Preformzuführung, einer Heizvorrichtung einer Blasmaschine, einem Rinser, einer Sterilisationseinrichtung, einer Behälterbeschichtungsstation, einem Füller, einem Verschließer und/oder einer Etikettiervorrichtung angeordnet sein. Der Messdummy kann durch die Behälterbehandlungsanlage oder durch Teile der Behälterbehandlungsanlage bewegt werden und dabei kann das Messelement den Wert wenigstens eines Parameter bestimmen. Dadurch kann eine Messung eines orts- und/oder zeitabhängigen Parameters durchgeführt werden.

Das wenigstens eine Messelement kann insbesondere derart ausgebildet sein, dass es eine Keimanzahl und/oder eine Masse oder Anzahl von Schmutzpartikeln messen kann. Das wenigstens eine Messelement kann außerdem derart ausgebildet sein, dass es einen Druckwert, einen Temperaturwert, einen Wert einer Kraft, einen Wert eines Moments, einen Positionswert, eine Zeitdauer, eine Gas- oder Flüssigkeitsmenge, insbesondere einer Sterilisationsflüssigkeit, zum Beispiel einen Volumenwert, eine Geschwindigkeit, eine Strahlungsstärke und/oder eine Feldstärke messen kann. Der Messdummy kann insbesondere mehrere Messelemente umfassen, wobei jedes der Messelemente einen oder mehrere der oben genannten Werte bestimmen oder messen kann.

Der Messdummy kann außerdem ein Sendeelement zum Senden des wenigstens einen vom Messelement bestimmten Parameterwertes an ein Auswerteelement umfassen.

Der Messdummy kann außerdem ein Speicherelement zum Speichern des wenigstens einen vom Messelement bestimmten Parameterwertes umfassen.

Der Messdummy kann außerdem eine Schnittstelle, insbesondere eine USB-Schnittstelle, zum Verbinden des Speicherelements mit einem Auswerteelement und/oder einem externen Speicherelement umfassen.

Der Messdummy kann außerdem ein Energieversorgungselement zum Versorgen des wenigstens einen Messelements, des Sendeelements, des Speicherelements und/oder der Schnittstelle mit elektrischer Energie umfassen.

Der Messdummy kann wenigstens teilweise durch eine Transportvorrichtung bewegt werden, mit welcher im Betrieb die zu bearbeitenden Artikel bewegt werden.

Der Messdummy kann außerdem derart ausgebildet und/oder konfiguriert sein, dass er den wenigstens einen vom Messelement bestimmten Parameterwertes an ein Auswerteelement übermittelt. Insbesondere kann die bestimmte Restverschmutzungsmenge an ein Auswerteelement übermittelt werden.

Der Testbehälter, insbesondere der Messdummy, kann außerdem wenigstens ein Element zum Reinigen von Transportelementen einer Transportvorrichtung der Behälterbehandlungsanlage, umfassen. Dadurch können Schmutzrückstände, welche sich an Transportelementen ablagern entfernt oder wenigstens minimiert werden wenn der Testbehälter mittels der Transportvorrichtung wenigstens teilweise durch die Behälterbehandlungsanlage bewegt wird.

Das wenigstens eine Element zum Reinigen von Transportelementen der Transportvorrichtung kann beispielsweise eine Büste sein.

Die Vorrichtung zum Überprüfen der Reinigungswirkung kann auch derart ausgebildet und/oder Konfiguriert sein, dass mehrere Testbehälter zum Überprüfen der Reinigungswirkung der Reinigungsvorrichtung verwendet werden. Mehrere Testbehälter können insbesondere mit unterschiedlichen vorherbestimmten Verschmutzungsmengen und/oder Verschmutzungsarten versehen werden. Alternativ oder zusätzlich kann die vorherbestimmte Verschmutzungsmenge für unterschiedliche Testbehälter in unterschiedlichen Teilbereichen angeordnet werden. Dadurch kann die Reinigungswirkung für unterschiedliche Arten und Grade der Verschmutzung überprüft werden.

Eine oben beschriebene Vorrichtung zum Überprüfen der Reinigungswirkung kann insbesondere Teil einer Behälterbehandlungsanlage sein. Mit anderen Worten stellt die Erfindung außerdem ein System umfassend eine Behälterbehandlungsmaschine und eine oben beschriebene Vorrichtung zum Überprüfen der Reinigungswirkung einer Reinigungsvorrichtung bereit, wobei die Reinigungsvorrichtung Teil der Behälterbehandlungsanlage ist.

Die Behälterbehandlungsmaschine und/oder die Vorrichtung zum Überprüfen der Reinigungswirkung der Reinigungsvorrichtung können ein oder mehrere der oben beschriebenen Merkmale umfassen.

Die Erfindung stellt außerdem ein Verfahren zum Überprüfen der Reinigungswirkung einer Reinigungsvorrichtung, wobei die Reinigungsvorrichtung Teil einer Behälterbehandlungsanlage ist, bereit, umfassend:
maschinelles Einbringen und/oder Aufbringen einer vorherbestimmten Verschmutzungsmenge in und/oder auf einen Testbehälter; und
maschinelles Einschleusen des Testbehälters in die Behälterbehandlungsanlage.

Dadurch dass das Einbringen und/oder Aufbringen der vorherbestimmten Verschmutzungsmenge und das Einschleusen des Testbehälters in die Behälterbehandlungsanlage maschinell durchgeführt wird, kann eine Kontamination des Testbehälters durch eine Bedienperson verhindert oder wenigstens minimiert werden, und dadurch eine bessere Überprüfung der Reinigungswirkung der Reinigungsvorrichtung ermöglicht werden.

Das Verfahren kann insbesondere mittels einer oben beschriebenen Vorrichtung durchgeführt werden.

Nach dem Einschleusen des Testbehälters in die Behälterbehandlungsanlage kann der Testbehälter durch ein oder mehrere Behandlungselemente der Behälterbehandlungsanlage behandelt werden, insbesondere in derselben Weise wie zu behandelnde Behälter im Betrieb der Behälterbehandlungsanlage.

Der Testbehälter kann insbesondere durch vorherbestimmte Behandlungselemente behandelt werden. Insbesondere kann auf ein Befüllen des Testbehälters verzichtet werden. Auf diese Weise kann beispielsweise die Keimvermehrung wie oben beschrieben gemessen werden. Prinzipiell wäre es aber auch möglich, die Keimvermehrung mit abgefülltem Produkt zu messen.

Das Verfahren umfasst insbesondere ein Reinigen des Testbehälters mit der Reinigungsvorrichtung und ein Bestimmen einer Restverschmutzungsmenge nach dem Reinigen des Testbehälters mit der Reinigungsvorrichtung.

Das Bestimmen der Restverschmutzungsmenge kann insbesondere automatisch, beispielsweise durch einen Sensor, erfolgen. Alternativ oder zusätzlich kann das Bestimmen der Restverschmutzungsmenge in einem Labor erfolgen.

Das Einschleusen des Testbehälters kann vor, nach und/oder während des Einbringens und/oder Aufbringens der vorherbestimmten Verschmutzungsmenge durchgeführt werden.

Das Verfahren kann außerdem ein Reinigen, insbesondere Sterilisieren, des Testbehälters vor dem des Einbringen und/oder Aufbringen der vorherbestimmten Verschmutzungsmenge umfassen. Dadurch kann eine noch bessere Überprüfung der Reinigungswirkung der Reinigungsvorrichtung ermöglicht werden, da eine eventuell vorhandene Verschmutzung des Testbehälters vor dem des Einbringen und/oder Aufbringen der vorherbestimmten Verschmutzungsmenge entfernt oder wenigstens minimiert wird.

Das Verfahren kann außerdem ein Ausschleusen des Testbehälters aus der Behälterbehandlungsanlage, insbesondere vor, nach und/oder während des Bestimmens der Restverschmutzungsmenge, umfassen.

Das Verfahren kann außerdem ein Vergleichen der bestimmten Restverschmutzungsmenge mit einem vorherbestimmten Wert, insbesondere mit der vorherbestimmten Verschmutzungsmenge umfassen. Dadurch kann die Reinigungswirkung der Reinigungsvorrichtung quantifiziert werden.

Insbesondere kann die bestimmte Restverschmutzungsmenge mit einem vorherbestimmten Wert verglichen werden. Dadurch kann die Reinigungswirkung der Reinigungsvorrichtung klassifiziert werden.

Das Vergleichen kann insbesondere automatisch, beispielsweise durch ein Steuerungselement, durchgeführt werden. Wenn der Vergleich der bestimmten Restverschmutzungsmenge mit dem vorherbestimmten Wert ein vorherbestimmtes Kriterium nicht erfüllt, kann beispielsweise eine Ausgabe an eine Bedienperson erfolgen, beispielsweise in Form eines optisches und/oder akustisches Warnsignals. Dadurch kann beispielsweise eine Bedienperson benachrichtigt werden, wenn die Restverschmutzung einen zu hohen Wert annimmt.

Die vorherbestimmte Verschmutzungsmenge kann an einer oder mehreren vorherbestimmten Stellen des Testbehälters angeordnet werden. Insbesondere kann die Verschmutzungsmenge beispielsweise an schwer reinigbaren Teilen des Behälters angeordnet werden. Die vorherbestimmte Verschmutzungsmenge kann beispielsweise am Boden und/oder an einem oder mehreren Gewinden oder Mundstücken des Testbehälters angeordnet werden.

Der Testbehälter kann einen oder mehrere Hinterschnitte aufweisen, insbesondere wobei die vorherbestimmte Verschmutzungsmenge wenigstens teilweise im Bereich des wenigstens einen Hinterschnitts aufgebracht und/oder eingebracht wird.

Das Verfahren umfasst außerdem ein Regeln wenigstens eines Betriebsparameters der Behälterbehandlungsanlage, insbesondere der Reinigungsvorrichtung, in Abhängigkeit von der bestimmten Restverschmutzungsmenge, insbesondere auf dem Ergebnis des Vergleichs der bestimmten Restverschmutzungsmenge mit dem vorherbestimmten Wert. Dadurch kann die Reinigungswirkung der Reinigungsvorrichtung gesteuert oder geregelt werden. Insbesondere kann dadurch der Betrieb und/oder Materialeinsatz der Reinigungsvorrichtung optimiert werden.

Insbesondere kann ein Betriebsparameter wenigstens eines Reinigungselements der Reinigungsvorrichtung basierend auf der bestimmten Restverschmutzungsmenge, insbesondere auf dem Ergebnis des Vergleichs der bestimmten Restverschmutzungsmenge mit dem vorherbestimmten Wert, eingestellt oder geregelt werden.

Beispielsweise kann die Menge, die Konzentration und/oder die Temperatur einer Sterilisationsflüssigkeit und/oder der Einspritz- oder Blasdruck der Sterilisationsflüssigkeit eingestellt, gesteuert oder geregelt werden. Alternativ oder zusätzlich kann eine Einstellung, Steuerung oder eine Regelung einer Menge einer Sterilisationsflüssigkeit, einer Position am Testbehälter für die Aufbringung der vorherbestimmten Verschmutzungsmenge, z.B. über eine Eintauchtiefe einer Lanze, einer Strahlungsstärke einer UV-Lampe und/oder einer Mikrowelleneinrichtung, einer Art Sterilisationsmedium für die Behälter, beispielsweise Wasserstoffperoxid oder Peressigsäure oder Seifenlauge, einer verwendeten Sterilisationseinrichtung, einer Reinigungszeit für die Vorrichtungen und/oder Behälter, einem Blasdruck einer Sterilisationsflüssigkeit und/oder einem Umgebungsdruck in der Behälterbehandlungsanlage oder in einem Teil der Behälterbehandlungsanlage durchgeführt werden.

Das Verfahren kann außerdem ein Reinigen von einem oder mehreren Elementen der Anlage, die von dem Testbehälter durchlaufen wurden, umfassen. Dadurch kann die Sterilität der Maschinen in einer nachfolgenden Produktion gewährleistet werden. Insbesondere können Elemente der Anlage nach jedem Durchlauf eines mit einer vorherbestimmten Verschmutzungsmenge versehenen Testbehälters gereinigt werden, insbesondere durch ein CIP (cleaning in place) Verfahren. Beispielsweise können Heizdorne, ein Ventilblock, der Sterilraum, Transportelemente und/oder Füllventile gereinigt werden.

Das Einschleusen und/oder Ausschleusen kann insbesondere derart durchgeführt werden, dass keine zusätzliche Verschmutzung in und/oder auf den Testbehälter eingebracht und/oder aufgebracht wird. Dadurch kann eine zusätzliche Kontamination des Testbehälters verhindert werden.

Bei dem Testbehälter kann es sich um einen im Betrieb der Behälterbehandlungsmaschine verwendbaren Vorformling oder Behälter, beispielsweise eine Flasche, handeln. Dadurch kann das Verfahren mit geringem Aufwand durchgeführt werden.

Alternativ kann der Testbehälter einem oben beschriebenen Messdummy entsprechen.

Das oben beschriebene Verfahren kann insbesondere mit mehr als einem Testbehälter durchgeführt werden. Insbesondere können wenigstens zwei voneinander unterschiedliche Testbehälter verwendet werden. Dadurch können unterschiedliche Teile der Behälterbehandlungsanlage und darin angeordnete Reinigungselemente überprüft werden. Beispielsweise kann ein Testbehälter in Form einer Flasche ausgebildet sein und ein weiterer Testbehälter in Form eines Vorformlings.

Die wenigstens zwei voneinander unterschiedlichen Testbehälter können insbesondere hintereinander maschinell mit einer vorherbestimmten Verschmutzungsmenge versehen werden und hintereinander maschinell in die Behälterbehandlungsanlage eingeschleust werden.

Mehrere Testbehälter können insbesondere mit unterschiedlichen vorherbestimmten Verschmutzungsmengen und/oder Verschmutzungsarten versehen werden. Alternativ oder zusätzlich kann die vorherbestimmte Verschmutzungsmenge für unterschiedliche Testbehälter in unterschiedlichen Teilbereichen angeordnet werden. Dadurch kann die Reinigungswirkung für unterschiedliche Arten und Grade der Verschmutzung überprüft werden.

Das Einschleusen und/oder Ausschleusen des einen oder der mehreren Testbehälter kann an oben beschriebenen Stellen der Behälterbehandlungsanlage durchgeführt werden. Insbesondere kann der wenigstens eine Testbehälter vor, insbesondere unmittelbar vor, der Reinigungsvorrichtung, insbesondere einem vorherbestimmten Reinigungselement der Reinigungsvorrichtung, eingeschleust werden und nach, insbesondere unmittelbar nach, der Reinigungsvorrichtung, insbesondere einem vorherbestimmten Reinigungselement der Reinigungsvorrichtung, ausgeschleust werden. Dadurch kann die Reinigungswirkung der Reinigungsvorrichtung bestimmt werden.

Ein oder mehrere Schritte eines oben beschriebenen Verfahrens können auch mehrfach, insbesondere wiederholt, durchgeführt werden. Beispielsweise kann wenigstens ein Testbehälter an unterschiedlichen Stellen in die Behälterbehandlungsanlage eingeschleust werden und/oder an unterschiedlichen Stellen aus der Behälterbehandlungsanlage ausgeschleust werden. Dadurch kann die Reinigungswirkung von Reinigungselementen der Reinigungsvorrichtung in unterschiedlichen Abschnitten der Behälterbehandlungsanlage überprüft werden.

Insbesondere kann das Verfahren in, je nach Anforderung, vorherbestimmten Intervallen, insbesondere zwischen zwei Produktionsprozessen, durchgeführt werden. Beispielsweise kann ein Intervall eine halbe oder volle Stunde, vier, acht, zwölf oder 24 Stunden, aber auch eine Woche bis zu einem Monat betragen. Alternativ oder zusätzlich kann das Verfahren in unregelmäßigen zeitlichen Abständen, zum Beispiel zwischen der Abfüllung zweier verschiedener Produkte auf einer Abfülllinie, durchgeführt werden.

Insbesondere können bei diesem Verfahren die Testbehälter zur Nachverfolgung, für Laborzwecke beispielsweise, mit zusätzlichen Identifizierungsmerkmalen wie RFID-Chips und/oder Strichcodes versehen werden. Insbesondere eignet sich die Identifizierung beim Aufbringen unterschiedlicher Verschmutzungsmengen auf mehrere Testbehälter, auch um mehrere Reinigungsvorrichtungen oder -elemente zu vergleichen.

Weitere Vorteile und Merkmale der Erfindung werden nachfolgend anhand der beispielhaften Figuren erläutert. Dabei zeigt
- Figur 1: eine Illustration eines beispielhaften Verfahrens zum Überprüfen der Reinigungswirkung einer Reinigungsvorrichtung in Form eines Flussdiagramms;
- Figur 2: eine beispielhafte Behälterbehandlungsanlage mit einer Reinigungsvorrichtung;
- Figur 3: eine weitere beispielhafte Behälterbehandlungsanlage mit einer Reinigungsvorrichtung;
- Figur 4: eine weitere beispielhafte Behälterbehandlungsanlage mit einer Reinigungsvorrichtung;
- Figur 5: eine beispielhafte Behälterbehandlungsanlage mit einer Vorrichtung zum Überprüfen der Reinigungswirkung einer Reinigungsvorrichtung;
- Figur 6: eine beispielhafte Einrichtung zum Einschleusen eines Testbehälters;
- Figur 7: eine weitere beispielhafte Behälterbehandlungsanlage mit einer Vorrichtung zum Überprüfen der Reinigungswirkung einer Reinigungsvorrichtung;
- Figur 8: einen beispielhaften Testbehälter;
- Figur 9: eine beispielhafte Einrichtung zum Einbringen und/oder Aufbringen einer vorherbestimmten Verschmutzungsmenge in und/oder auf einen Testbehälter; und
- Figur 10: eine weitere beispielhafte Einrichtung zum Einbringen und/oder Aufbringen einer vorherbestimmten Verschmutzungsmenge in und/oder auf einen Testbehälter.

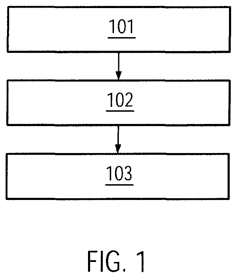

Figur 1 illustriert ein beispielhaftes Verfahren zum Überprüfen der Reinigungswirkung einer Reinigungsvorrichtung in Form eines Flussdiagramms. Die Reinigungsvorrichtung ist dabei Teil einer Behälterbehandlungsanlage, beispielsweise einer Abfüllanlage für Getränke.

Zunächst wird in einem ersten Schritt 101 eine vorherbestimmte Verschmutzungsmenge, beispielsweise eine vorherbestimmte Anzahl an Keimen und/oder Schmutzpartikeln, maschinell in und/oder auf einen Testbehälter eingebracht und/oder aufgebracht.

Die vorherbestimmte Verschmutzungsmenge kann beispielsweise 100.000 bis 1 Million Sporen von Bakterien in einer Suspension von 10 bis 100 Mikroliter bis hin zu 10 Milliliter Flüssigkeit entsprechen.

Bei dem Testbehälter kann es sich beispielsweise um einen Behälter handeln, für dessen Reinigung die Reinigungsvorrichtung vorgesehen ist. Beispielsweise kann es sich bei dem Testbehälter um eine Flasche, insbesondere ein Kunststoffflasche, handeln. Bei dem Testbehälter kann es sich alternativ auch um einen Messdummy handeln. Der Messdummy kann beispielsweise ein Messelement zur Bestimmung der Verschmutzungsmenge, beispielsweise einer Keimanzahl oder einer Anzahl an Schmutzpartikeln, und/oder zusätzliche Hinterschnitte, umfassen.

Der Testbehälter wird dann in einem Schritt 102 maschinell in die Behälterbehandlungsanlage eingeschleust oder eingebracht. Dadurch dass das Einbringen und/oder Aufbringen der vorherbestimmten Verschmutzungsmenge und das Einschleusen des Testbehälters in die Behälterbehandlungsanlage maschinell durchgeführt wird, kann eine Kontamination des Testbehälters durch eine Bedienperson verhindert oder wenigstens minimiert werden, und dadurch eine bessere Überprüfung der Reinigungswirkung der Reinigungsvorrichtung ermöglicht werden.

In diesem Beispiel wird der Testbehälter zunächst gezielt mit einer definierten Verschmutzungsmenge versehen und danach in die Behälterbehandlungsanlage eingeschleust. Alternativ kann der Schritt des Einbringens und/oder Aufbringens einer vorherbestimmten Verschmutzungsmenge aber auch während und/oder nach dem maschinellen Einschleusen des Testbehälters in die Behälterbehandlungsanlage durchgeführt werden.

In Schritt 103 wird eine Restverschmutzungsmenge in und/oder auf dem Testbehälter bestimmt. Das Bestimmen der Restverschmutzungsmenge kann vor, nach und/oder während eines Ausschleusens des Testbehälters aus der Behälterbehandlungsanlage durchgeführt werden.

Wenigstens ein Betriebsparameter der Behälterbehandlungsanlage, insbesondere der Reinigungsvorrichtung, kann in Abhängigkeit von der bestimmten Restverschmutzungsmenge geregelt werden. Dadurch kann die Effizienz der Reinigungsmaschine verbessert werden.

Wenn beispielsweise bestimmt wird, dass die bestimmte Restverschmutzungsmenge größer als ein vorherbestimmter Schwellenwert ist, kann die Reinigungswirkung der Reinigungsvorrichtung erhöht werden, indem wenigstens ein Betriebsparameter der Reinigungsvorrichtung und/oder der Behälterbehandlungsanlage angepasst wird. Wenn dagegen bestimmt wird, dass die bestimmte Restverschmutzungsmenge kleiner als ein vorherbestimmter Schwellenwert ist, kann die Reinigungswirkung der Reinigungsvorrichtung auch erniedrigt werden, wodurch beispielsweise der Verbrauch an Reinigungsmitteln in der Reinigungsvorrichtung reduziert werden kann.

Der wenigstens eine Betriebsparameter kann einer Konzentration einer Sterilisationsflüssigkeit, einer Menge einer Sterilisationsflüssigkeit, einer Temperatur einer Sterilisationsflüssigkeit, einem Einspritzdruck einer Sterilisationsflüssigkeit, einem Blasdruck einer Sterilisationsflüssigkeit und/oder einem Umgebungsdruck in der Behälterbehandlungsanlage oder in einem Teil der Behälterbehandlungsanlage entsprechen.

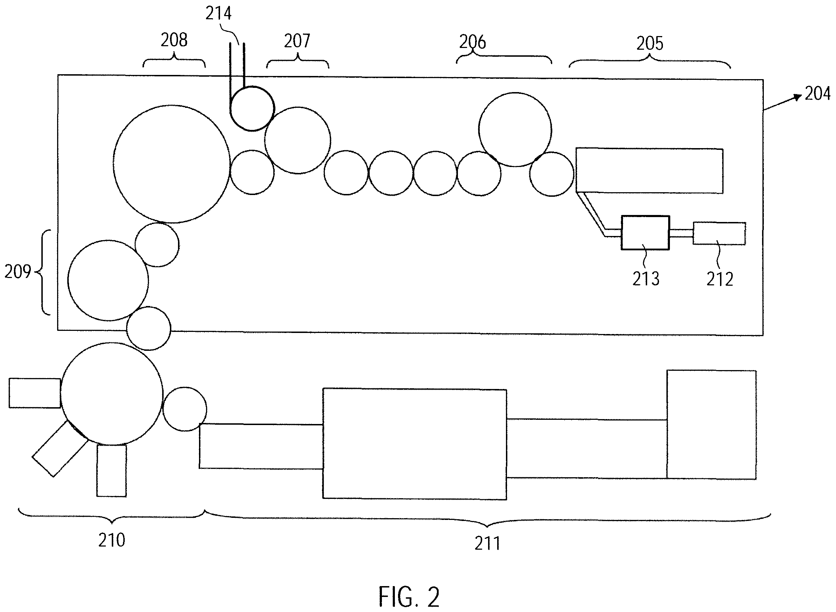

Figuren 2 bis 4 zeigen eine beispielhafte Behälterbehandlungsanlage, insbesondere eine Abfüllanlage, umfassend eine Heizvorrichtung für Vorformlinge 205, 305, 405 ein Blasmodul 206, 306, 406 ein Reinigungselement 207, 307, 407 einen Füller 208, 308, 408 und einen Verschließer 209, 309, 409 die in einem Reinraum 204, 304, 404 angeordnet sind. Die Behälterbehandlungsanlage umfasst außerdem eine Etikettiervorrichtung 210, 310, 410 und eine Packvorrichtung 211, 311, 411. Kunststoffvorformlinge, aus denen im Blasmodul 206, 306, 406 Kunststoffbehälter mittels Blasen, insbesondere Streckblasen, geformt werden, werden über eine Preformzuführung 212, 312, 412 in die Heizvorrichtung 205, 305, 405 eingebracht.

In den Figuren 2 bis 4 ist außerdem eine Vorrichtung zum Überprüfen der Reinigungswirkung einer Reinigungsvorrichtung der Behälterbehandlungsanlage, insbesondere des Reinigungselements 207, 307, 407, gezeigt.

Im Bereich der Preformzuführung 212, 312, 412 ist eine Einrichtung 213, 413, 513 zum Einbringen und/oder Aufbringen einer vorherbestimmten Verschmutzungsmenge in und/oder auf einen Testbehälter angeordnet. Insbesondere können mit der Einrichtung 213, 413, 513 ein oder mehrere Preforms mit einer vorherbestimmten Verschmutzungsmenge versehen werden.

In diesem Beispiel werden also Vorformlinge als Testbehälter verwendet. Alternativ kann auch wenigstens ein Messdummy als Testbehälter verwendet werden. Außerdem entspricht in diesem Beispiel die Einrichtung zum Einschleusen des Testbehälters der Preformzuführung 212, 312, 412. Es kann die Einrichtung zum Einschleusen des Testbehälters jedoch auch als separates Element vorgesehen sein.

Zusätzlich zu dem Reinigungselement 207, 307, 407 können noch weitere Reinigungselemente in der Behälterbehandlungsanlage vorgesehen sein.

Figuren 2 bis 4 zeigen außerdem Einrichtungen 214, 314, 414 zum Ausschleusen des Testbehälters aus der Behälterbehandlungsanlage.

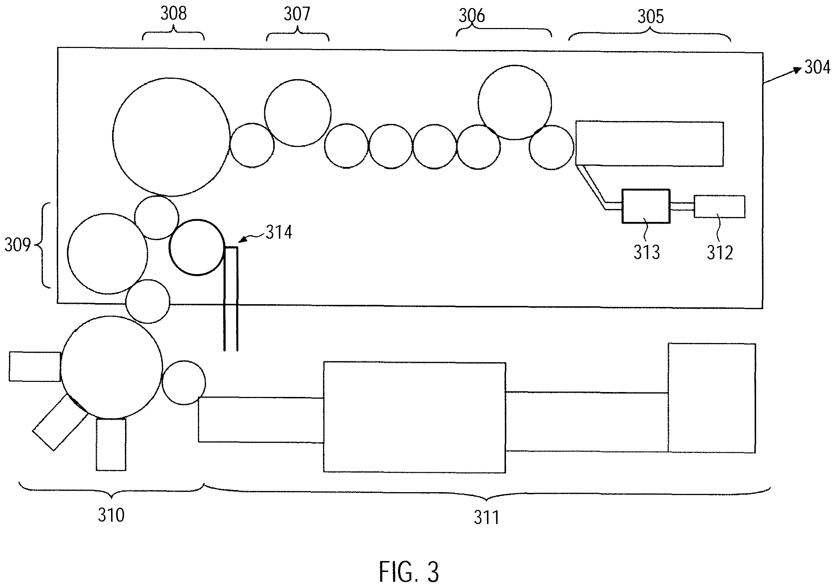

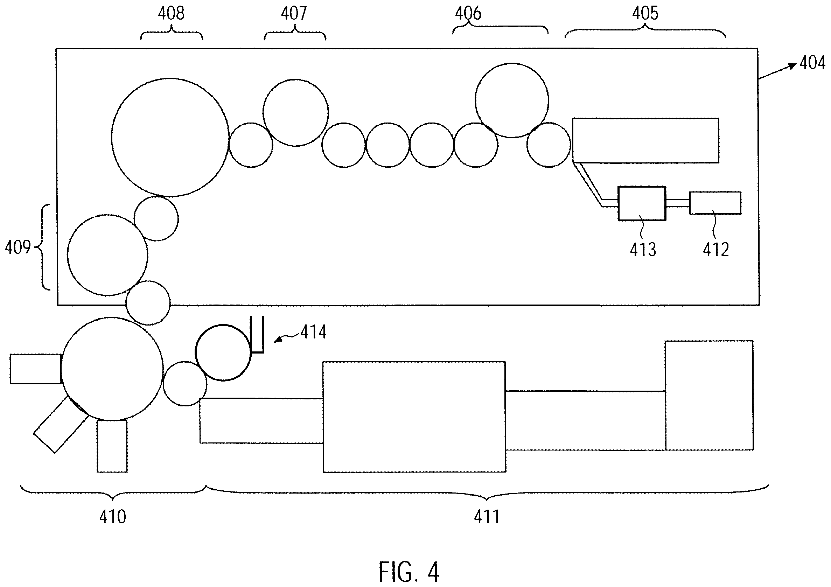

In Figur 2 ist die Einrichtung 214 zum Ausschleusen des Testbehälters unmittelbar nach der Reinigungselement 207 angeordnet. In Figur 3 ist die Einrichtung 314 zum Ausschleusen des Testbehälters alternativ nach dem Füller 308 angeordnet. In Figur 4 ist die Einrichtung zum Ausschleusen des Testbehälters 414 nach der Etikettiervorrichtung vorgesehen.

Durch die unterschiedliche Anordnung der Einrichtung 214, 314, 414 zum Ausschleusen des Testbehälters kann die Reinigungswirkung unterschiedlicher oder mehrerer Reinigungselemente der Behälterbehandlungsanlage überprüft werden, beispielsweise Reinigungselemente des Blasmoduls 206, 306, 406 des Füllers 208, 308, 408 oder der Etikettiervorrichtung 210, 310, 410. Diese zusätzlichen Reinigungselemente sind in den Figuren 2 bis 4 nicht dargestellt.

Die Vorrichtung zum Überprüfen der Reinigungswirkung kann außerdem Sensoren umfassen, mit denen eine Restverschmutzungsmenge in und/oder auf dem Testbehälter bestimmt werden kann. Dadurch kann eine weitere direkte Interaktion des Testbehälters mit einer Bedienperson zum Bestimmen der Restverschmutzungsmenge vermieden werden, wodurch eine weitere mögliche Kontamination oder Verschmutzung des Testbehälters vermieden oder wenigstens reduziert werden kann.

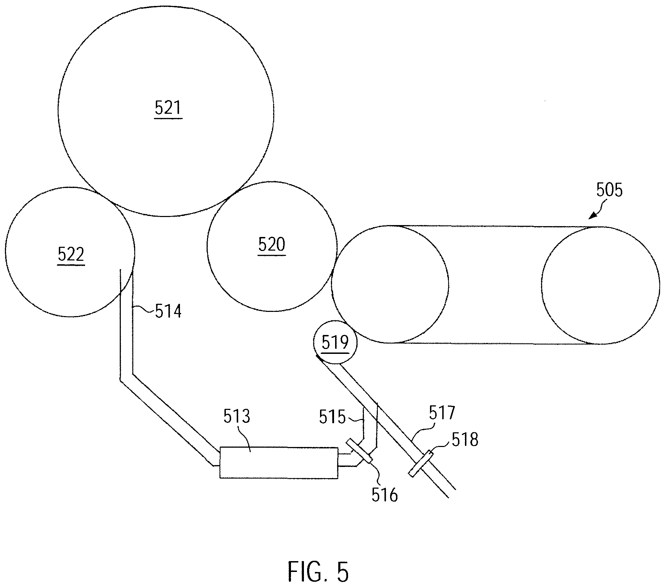

Figur 5 zeigt eine beispielhafte Behälterbehandlungsanlage in Form einer Blasmaschine und eine Vorrichtung zum Überprüfen der Reinigungswirkung einer Reinigungseinrichtung der Blasmaschine, beispielsweise der Heizvorrichtung oder eines nicht dargestellten Preformrinsers.

In die beispielhafte Blasmaschine können Behälter über ein Einlaufrad 519 eingeschleust werden. Zum Einen können Vorformlinge zum Herstellen von Kunststoffbehältern über eine Einrichtung 517 in die Blasmaschine eingebracht werden. Zum Anderen können Testbehälter in einer Einrichtung 513 zum Einbringen und/oder Aufbringen einer vorherbestimmten Verschmutzungsmenge in und/oder auf einen Testbehälter mit einer vorherbestimmten Verschmutzungsmenge versehen werden und über eine Einrichtung 515 zum Einschleusen des Testbehälters in die Blasmaschine eingebracht werden. Durch Stopper 516 beziehungsweise 518 können die Transportwege für die Zuführung von Vorformlingen beziehungsweise Testbehälter, insbesondere temporär, gesperrt werden.

Die Blasmaschine umfasst außerdem Transferelemente 520 bzw. 522.

Nach Durchlauf eines Testbehälters durch die Heizvorrichtung 505 und das Blasrad 521 kann der Testbehälter durch eine Einrichtung 514 zum Ausschleusen des Testbehälters aus der Anlage ausgeschleust werden.

Der Testbehälter kann beispielsweise ein Messdummy sein, der ein Messelement zum Bestimmen einer Restverschmutzungsmenge umfasst. Die bestimmte Restverschmutzungsmenge kann durch Verbinden eines Anschlusses des Messdummys mit Kontakten im Rückförderweg zur Einrichtung 513 zum Einbringen und/oder Aufbringen einer vorherbestimmten Verschmutzungsmenge in und/oder auf einen Testbehälter ausgelesen werden oder über ein Sendeelement des Messdummys, beispielsweise einen RFID-Chip (Radio Frequency Identity-Chip), einen Infrarot Sender oder einen "Bluetooth" Sender, an ein Auswerteelement, beispielsweise einen Personal Computer (PC) oder eine Speicherprogrammierbare Steuerung (SPS), gesendet werden.

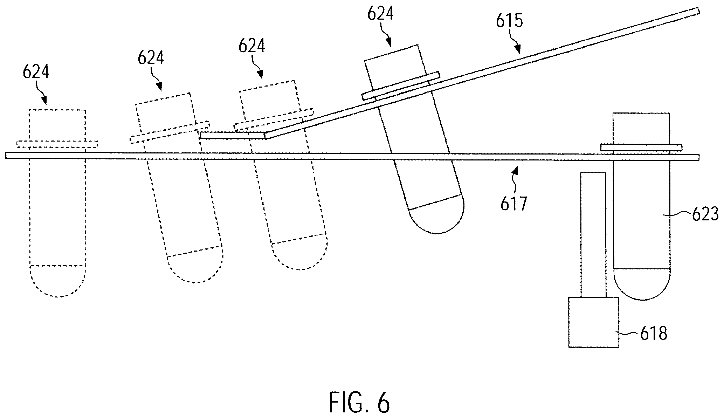

Figur 6 zeigt eine beispielhafte Einrichtung 615 zum Einschleusen des Testbehälters in eine Behälterbehandlungsanlage. Insbesondere können mehrere Testbehälter 624 in einer Sequenz in die Behälterbehandlungsanlage eingebracht werden. Dazu kann ein Stopper 618 vorgesehen sein, welcher den Fluss von Vorformlingen 623 unterbricht, welche über eine Einrichtung 617 in die Behälterbehandlungsanlage eingebracht werden können.

Die Testbehälter 624 können beispielsweise unterschiedliche vorherbestimmte Verschmutzungsmengen aufweisen und/oder in unterschiedlichen Teilbereichen mit einer vorherbestimmte Verschmutzungsmenge versehen sein. Dadurch kann die Reinigungswirkung für unterschiedliche Arten und Grade der Verschmutzung überprüft werden.

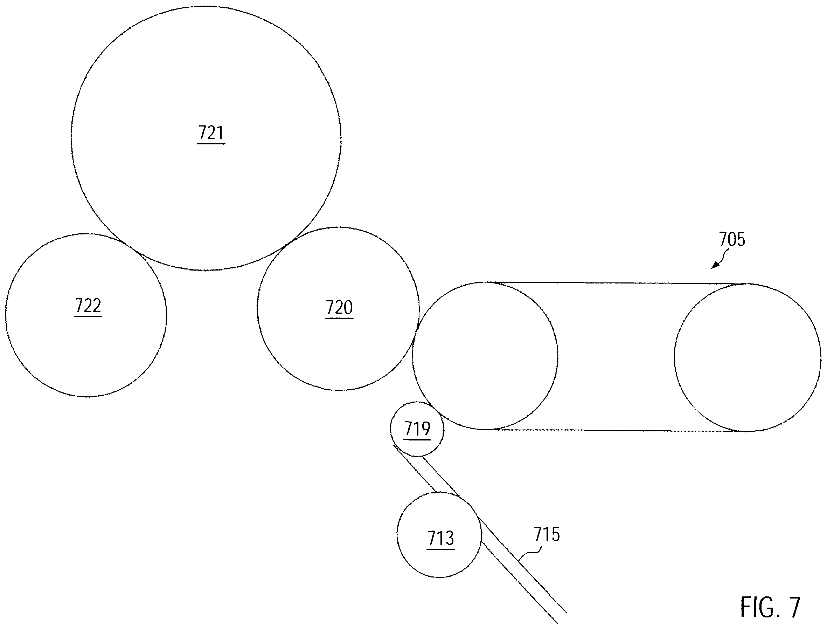

Figur 7 zeigt eine weitere beispielhafte Behälterbehandlungsanlage in Form einer Blasmaschine. Die Blasmaschine entspricht im Wesentlichen der Blasmaschine aus Figur 5 und umfasst ein Einlaufrad 719, eine Heizvorrichtung 705, ein Blasrad 721 und Transferelemente 720 und 722. Die Einrichtung 713 zum Einbringen und/oder Aufbringen einer vorherbestimmten Verschmutzungsmenge in und/oder auf einen Testbehälter ist in diesem Beispiel als Rundläufer ausgebildet.

Die Einrichtung 713 kann auch gleichzeitig ein Preformrinser sein, der Vorformlinge mit ionisierter Luft oder mit einem Reinigungsmedium ausspülen kann. Die Einrichtung 713 kann derart ausgebildet und/oder konfiguriert sein, dass nach dem Einbringen und/oder Aufbringen einer vorherbestimmten Verschmutzungsmenge in und/oder auf einen Testbehälter eine Selbstreinigung (Cleaning in Place, CIP) der Einrichtung 713 durchgeführt wird. Damit kann eine Kontamination von Vorformlingen bei einem anschließenden Reinigen der Vorformlinge verhindert oder wenigstens minimiert werden.

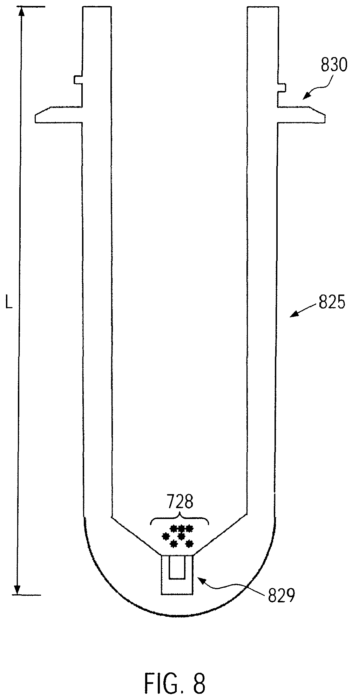

Ein beispielhafter Testbehälter in Form eines Messdummys 825 ist in Figur 8 gezeigt. In dem beispielhaften Messdummy 825 ist eine vorherbestimmte Anzahl an Keimen 828 eingebracht, welche insbesondere am Boden des Messdummys 825 angeordnet ist. Außerdem umfasst der beispielhafte Messdummy 825 ein Messelement 829 zum Bestimmen einer Anzahl an Keimen.

Der beispielhafte Messdummy 825 umfasst außerdem ein Mundstück 830. Im Bereich des Mundstücks 830 kann die vorherbestimmte Verschmutzungsmenge ganz oder teilweise angeordnet werden. Das Mundstück 830 umfasst insbesondere einen Tragring, einen Stützring und ein Gewinde.

Der beispielhafte Messdummy 825 kann in seiner Form und seinen geometrischen Abmessungen einem Vorformling entsprechen. Alternativ kann die Länge des Messdummys 825 der Länge eines herzustellenden Kunststoffbehälters entsprechen.

Im Bereich des Mundstücks 830 kann der Messdummy 825 auch wenigstens eine Bürste umfassen. Dadurch können Transportelemente der Behälterbehandlungsanlage, die mit den Bürsten in Kontakt kommen, gereinigt werden.

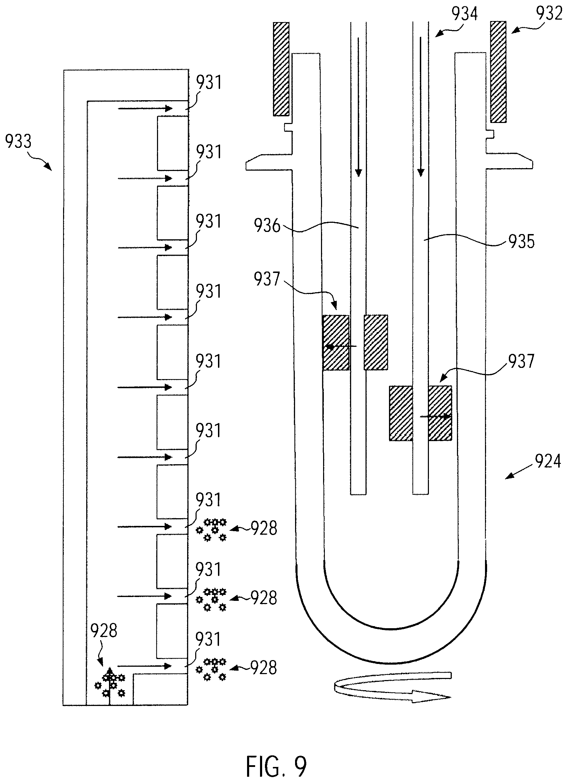

Figur 9 zeigt eine beispielhafte Einrichtung zum Einbringen und/oder Aufbringen einer vorherbestimmten Verschmutzungsmenge in und/oder auf einen Testbehälter 924 umfassend ein erstes Element 933 und ein zweites Element 934. Der Testbehälter 924 wird dabei von einem Außengreifer 932 gehaltert und kann dadurch gedreht werden, wie in der Figur durch einen Pfeil unter dem Testbehälter 924 schematisch angedeutet.

Das erste Element 933 der beispielhaften Einrichtung zum Einbringen und/oder Aufbringen einer vorherbestimmten Verschmutzungsmenge dient zum Aufbringen einer Verschmutzung auf dem Testbehälter 924, insbesondere auf einer Oberfläche der Außenseite des Testbehälters 924. Insbesondere wird hier eine vorherbestimmte Verschmutzungsmenge auf den Bereich des Mundstücks von außen aufgetragen, da dieser Bereich höhere Anforderungen bezüglich der Reinigung aufweist.

Das zweite Element 934 der beispielhaften Einrichtung zum Einbringen und/oder Aufbringen einer vorherbestimmten Verschmutzungsmenge dient zum Einbringen einer Verschmutzung in den Testbehälter 924, insbesondere auf eine Oberfläche der Innenseite des Testbehälters 924.

Das erste Element 933 umfasst eine Vielzahl von Düsen 931, über die eine Verschmutzung entlang der Längsachse des Testbehälters 924 auf dem Testbehälter 924 aufgebracht werden kann. Beispielsweise können Keime 928 in einer Suspension mittels Druckluft durch die Düsen 931 auf den Testbehälter 924 gesprüht werden.

Das zweite Element 934 ist in Form einer in den Testbehälter 924 eintauchenden Lanze ausgebildet. Die Lanze umfasst zwei Kanäle 935 und 936, an denen jeweils Kontaktelemente 937 angeordnet sind. Die Kontaktelemente 937 können derart ausgebildet sein, dass sie eine in den Kanälen 935 bzw. 936 eingebrachte Verschmutzungsmenge wenigstens teilweise aufnehmen können und diese durch Abrollen oder Abstreifen an einer Oberfläche des Testbehälters 925 anordnen können. Dafür können die Kontaktelemente 937 ein poröses oder schwammartiges Material umfassen. Über einen vorgegebenen Anpressdruck vom porösen Material zur Innenseite des Testbehälters kann so eine vorherbestimmte Menge an Verschmutzung aufgetragen werden.

Die Zuleitung der Verschmutzungsmenge in die Kanäle und deren Ausgänge kann entweder intervallartig, beispielsweise kurz vor der Auftragung, als auch mit konstantem Volumenstrom erfolgen.

Das erste Element 933 kann auch ein oder mehrere Kontaktelemente anstelle der oder zusätzlich zu den Düsen 931 umfassen.

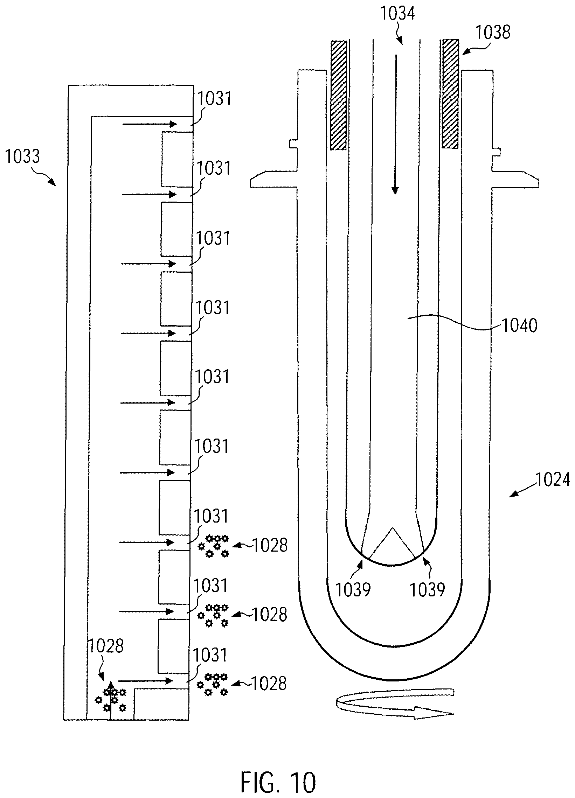

Figur 10 zeigt eine weitere beispielhafte Einrichtung zum Einbringen und/oder Aufbringen einer vorherbestimmten Verschmutzungsmenge in und/oder auf einen Testbehälter 1024 umfassend ein erstes Element 1033 und ein zweites Element 1034. Der Testbehälter 1024 wird dabei von einem Haltedorn 1038 gehaltert und kann mittels des Haltedorns 1038 auch gedreht werden, wie in der Figur durch einen Pfeil unter dem Testbehälter schematisch angedeutet. Alternativ können auch das erste Element 1033 und/oder das zweite Element 1034 drehbar sein. Denkbar wäre auch ein stationäres Einbringen ohne Drehung eines der Elemente.

Das erste Element 1033 entspricht im Wesentlichen dem ersten Element 933 in Figur 9. Insbesondere umfasst das erste Element 1033 eine Vielzahl an Düsen 1031.

Das zweite Element 1034 der beispielhaften Einrichtung zum Einbringen und/oder Aufbringen einer vorherbestimmten Verschmutzungsmenge dient zum Einbringen einer Verschmutzung in den Testbehälter 1025, insbesondere auf eine Oberfläche der Innenseite des Testbehälters 1025.

Das zweite Element 1034 ist in Form einer in den Testbehälter 1024 eintauchenden Lanze ausgebildet. Die Lanze umfasst mehrere Düsen 1039 mittels welcher eine Verschmutzung in den Testbehälter eingebracht werden kann. Dafür umfasst das zweite Element 1034 außerdem einen Kanal 1040, über den die Verschmutzung zu den Düsen 1039 geleitet wird. Die Verschmutzung kann dabei insbesondere in Form einer Suspension bereitgestellt werden.

Es versteht sich, dass in den zuvor beschriebenen Ausführungsbeispielen genannte Merkmale nicht auf diese speziellen Kombinationen beschränkt sind und auch in beliebigen anderen Kombinationen möglich sind.

## Patentansprüche

1. Vorrichtung zum Überprüfen der Reinigungswirkung einer Reinigungsvorrichtung, wobei die Reinigungsvorrichtung Teil einer Behälterbehandlungsanlage ist, umfassend:
eine Einrichtung (213; 313; 413; 513; 713) zum Einbringen und/oder Aufbringen einer vorherbestimmten Verschmutzungsmenge in und/oder auf einen Testbehälter (624; 825; 924; 1024);
eine Einrichtung (515; 715) zum Einschleusen des Testbehälters (624; 825; 924; 1024) in die Behälterbehandlungsanlage;
**dadurch gekennzeichnet, dass** die Vorrichtung eine Einrichtung zum Bestimmen einer Restverschmutzungsmenge nach der Reinigung des Testbehälters (624; 825; 924; 1024) mit der Reinigungsvorrichtung und
ein Steuerungselement, das derart konfiguriert ist, dass wenigstens ein Betriebsparameter der Behälterbehandlungsanlage, insbesondere der Reinigungsvorrichtung, in Abhängigkeit von der bestimmten Restverschmutzungsmenge geregelt wird, umfasst.

2. Vorrichtung nach Anspruch 1, außerdem umfassend eine Einrichtung (214; 314; 414; 514) zum Ausschleusen des Testbehälters (624; 825; 924; 1024) aus der Behälterbehandlungsanlage.

3. Vorrichtung nach Anspruch 1, wobei der wenigstens eine Betriebsparameter eine Konzentration einer Sterilisationsflüssigkeit, eine Menge einer Sterilisationsflüssigkeit, eine Temperatur einer Sterilisationsflüssigkeit, einem Einspritzdruck einer Sterilisationsflüssigkeit, einem Blasdruck einer Sterilisationsflüssigkeit und/oder einem Umgebungsdruck in der Behälterbehandlungsanlage entspricht.

4. Vorrichtung nach einem der vorangegangenen Ansprüche, wobei die Einrichtung (213; 313; 413; 513; 713) zum Einbringen und/oder Aufbringen der vorherbestimmten Verschmutzungsmenge ein Trocknungselement zum Trocknen des Testbehälters (624; 825; 924; 1024) umfasst.

5. Vorrichtung nach einem der vorangegangenen Ansprüche, wobei die Einrichtung (213; 313; 413; 513; 713) zum Einbringen und/oder Aufbringen der vorherbestimmten Verschmutzungsmenge ein Behandlungselement der Behälterbehandlungsanlage umfasst.

6. Vorrichtung nach einem der vorangegangenen Ansprüche, außerdem umfassend den Testbehälter (624; 825; 924; 1024), insbesondere wobei der Testbehälter (624; 825; 924; 1024) ein Messdummy ist.

7. System umfassend eine Behälterbehandlungsanlage und eine Vorrichtung nach einem der vorangegangenen Ansprüche.

8. Verfahren zum Überprüfen der Reinigungswirkung einer Reinigungsvorrichtung, wobei die Reinigungsvorrichtung Teil einer Behälterbehandlungsanlage ist, umfassend:
maschinelles Einbringen und/oder Aufbringen einer vorherbestimmten Verschmutzungsmenge in und/oder auf einen Testbehälter (624; 825; 924; 1024);
maschinelles Einschleusen des Testbehälters (624; 825; 924; 1024) in die Behälterbehandlungsanlage;
**dadurch gekennzeichnet, dass** das Verfahren das Bestimmen einer Restverschmutzungsmenge nach dem Reinigen des Testbehälters (624; 825; 924; 1024) mit der Reinigungsvorrichtung und das Regeln wenigstens eines Betriebsparameters der Behälterbehandlungsanlage, insbesondere der Reinigungsvorrichtung, in Abhängigkeit von der bestimmten Restverschmutzungsmenge umfasst.

9. Verfahren nach Anspruch 8, wobei das Einschleusen des Testbehälters (624; 825; 924; 1024) vor, nach und/oder während des Einbringen und/oder Aufbringen der vorherbestimmten Verschmutzungsmenge durchgeführt wird.

10. Verfahren nach einem der Ansprüche 8 - 9, außerdem umfassend Ausschleusen des Testbehälters (624; 825; 924; 1024) aus der Behälterbehandlungsanlage, insbesondere vor, nach und/oder während des Bestimmens der Restverschmutzungsmenge.

11. Verfahren nach einem der Ansprüche 8 - 10, außerdem umfassend Vergleichen der bestimmten Restverschmutzungsmenge mit einem vorherbestimmten Wert, insbesondere mit der vorherbestimmten Verschmutzungsmenge.

12. Verfahren nach Anspruch 11, wobei das Regeln wenigstens eines Betriebsparameters der Behälterbehandlungsanlage, insbesondere der Reinigungsvorrichtung, basierend auf dem Ergebnis des Vergleichs der bestimmten Restverschmutzungsmenge mit dem vorherbestimmten Wert durchgeführt wird.

13. Verfahren nach einem der Ansprüche 8 - 12, außerdem umfassend ein Reinigen von einem oder mehreren Elementen der Anlage, die von dem Testbehälter (624; 825; 924; 1024) durchlaufen wurden.

## Claims

1. A device for testing the cleaning effect of a cleaning apparatus, said cleaning apparatus being part of a container treatment plant, comprising:
a unit (213; 313; 413; 513; 713) for introducing and/or applying a predetermined amount of contamination in and/or to a test container (624; 825; 924; 1024);
a unit (515; 715) for introducing the test container (624; 825; 924; 1024) in the container treatment plant;
**characterized in that** the device comprises
a unit for determining an amount of residual contamination when the test container (624; 825; 924; 1024) has been cleaned by the cleaning apparatus and
a control element, which is configured such that at least one operating parameter of the container treatment plant, in particular of the cleaning apparatus, is controlled in dependence upon the determined amount of residual contamination.

2. A device according to claim 1, further comprising a unit (214; 314; 414; 514) for discharging the test container (624; 825; 924; 1024) from the container treatment plant.

3. A device according to claim 1, wherein the at least one operating parameter corresponds to a concentration of a sterilization liquid, an amount of a sterilization liquid, a temperature of a sterilization liquid, an injection pressure of a sterilization liquid, a blowing pressure of a sterilization liquid and/or an ambient pressure in the container treatment plant.

4. A device according to one of the preceding claims, wherein the unit (213; 313; 413; 513; 713) for introducing and/or applying a predetermined amount of contamination comprises a drying element for drying the test container (624; 825; 924; 1024).

5. A device according to one of the preceding claims, wherein the unit (213; 313; 413; 513; 713) for introducing and/or applying the predetermined amount of contamination comprises a treatment element of the container treatment plant.

6. A device according to one of the preceding claims, further comprising the test container (624; 825; 924; 1024), said test container (624; 825; 924; 1024) being in particular a measurement dummy.

7. A system comprising a container treatment plant and a device according to one of the preceding claims.

8. A method of testing the cleaning effect of a cleaning apparatus, wherein the cleaning apparatus is part of a container treatment plant, said method comprising:
automatic introduction and/or application of a predetermined amount of contamination in and/or to a test container (624; 825; 924; 1024);
automatic introduction of the test container (624; 825; 924; 1024) in the container treatment plant;
**characterized in that** the method comprises
determining an amount of residual contamination when the test container (624; 825; 924; 1024) has been cleaned by means of the cleaning apparatus and
controlling at least one operating parameter of the container treatment plant, in particular of the cleaning apparatus, in dependence upon the determined amount of residual contamination.

9. A method according to claim 8, wherein the test container (624; 825; 924; 1024) is introduced prior to, subsequent to and/or during the introduction and/or application of the predetermined amount of contamination.

10. A method according to one of the claims 8 - 9, further comprising discharging the test container (624; 825; 924; 1024) from the container treatment plant, in particular prior to, subsequent to and/or during the determination of the amount of residual contamination.

11. A method according to one of the claims 8 - 10, further comprising comparing the determined amount of residual contamination with a predetermined value, in particular with the predetermined amount of contamination.

12. A method according to claim 11, wherein the control of at least one operating parameter of the container treatment plant, in particular of the cleaning apparatus, is performed in dependence upon the result of the comparison between the determined amount of residual contamination and the predetermined value.

13. A method according to one of the claims 8 - 12, further comprising cleaning one or a plurality of elements of the plant through which the test container (624; 825; 924; 1024) has passed.

## Revendications

1. Equipement de vérification de l'effet nettoyant d'un dispositif de nettoyage, le dispositif de nettoyage faisant partie d'une installation de traitement de contenants, comprenant :
un dispositif (213; 313; 413; 513; 713) pour introduire et/ou appliquer un niveau d'encrassement prédéterminé dans et/ou sur un contenant de test (624; 825; 924; 1024) ;
un dispositif (515; 715) pour insérer le contenant de test (624; 825; 924; 1024) dans l'installation de traitement de contenants ;
**caractérisé en ce que** l'équipement comprend un dispositif de détermination d'un niveau d'encrassement résiduel après le nettoyage du contenant de test (624; 825; 924; 1024) à l'aide du dispositif de nettoyage, et un élément de commande, qui est configuré de manière telle, qu'au moins un paramètre de fonctionnement de l'installation de traitement de contenants, notamment du dispositif de nettoyage, soit régulé en fonction du niveau d'encrassement résiduel déterminé.

2. Equipement selon la revendication 1, comprenant en outre, un dispositif (214; 314; 414; 514) pour l'extration du contenant de test (624; 825; 924; 1024) hors de l'installation de traitement de contenants.

3. Equipement selon la revendication 1, dans lequel ledit au moins un paramètre de fonctionnement correspond à une concentration d'un liquide de stérilisation, une quantité d'un liquide de stérilisation, une température d'un liquide de stérilisation, une pression d'injection d'un liquide de stérilisation, une pression de soufflage d'un liquide de stérilisation et/ou une pression environnante dans l'installation de traitement de contenants.

4. Equipement selon l'une des revendications précédentes, dans lequel le dispositif (213; 313; 413; 513; 713) pour introduire et/ou appliquer ledit niveau d'encrassement prédéterminé comprend un élément de séchage pour sécher le contenant de test (624; 825; 924; 1024) .

5. Equipement selon l'une des revendications précédentes, dans lequel le dispositif (213; 313; 413; 513; 713) pour introduire et/ou appliquer ledit niveau d'encrassement prédéterminé comprend un élément de traitement de l'installation de traitement de contenants.

6. Equipement selon l'une des revendications précédentes, comprenant, en outre, le contenant de test (624; 825; 924; 1024), le contenant de test (624; 825; 924; 1024) étant notamment un objet de mesure factice.

7. Système comprenant une installation de traitement de contenants et un équipement selon l'une des revendications précédentes.

8. Procédé de vérification de l'effet nettoyant d'un dispositif de nettoyage, le dispositif de nettoyage faisant partie d'une installation de traitement de contenants, le procédé comprenant :
l'introduction et/ou l'application automatique mécanisée d'un niveau d'encrassement prédéterminé dans et/ou sur un contenant de test (624; 825; 924; 1024) ;
l'insertion automatique mécanisée du contenant de test (624; 825; 924; 1024) dans l'installation de traitement de contenants ;
**caractérisé en ce que** le procédé comprend
la détermination d'un niveau d'encrassement résiduel après le nettoyage du contenant de test (624; 825; 924; 1024) à l'aide d'un dispositif de nettoyage, et
la régulation d'au moins un paramètre de fonctionnement de l'installation de traitement de contenants, notamment du dispositif de nettoyage, en fonction du niveau d'encrassement résiduel déterminé.

9. Procédé selon la revendication 8, d'après lequel l'insertion du contenant de test (624; 825; 924; 1024) est effectuée avant, après et/ou pendant l'introduction et/ou l'application du niveau d'encrassement prédéterminé.

10. Procédé selon l'une des revendications 8 - 9, comprenant, en outre, l'extraction du contenant de test (624; 825; 924; 1024) de l'installation de traitement de contenants, notamment avant, après et/ou pendant la détermination du niveau d'encrassement résiduel.

11. Procédé selon l'une des revendications 8 - 10, comprenant, en outre, la comparaison du niveau d'encrassement résiduel à une valeur prédéterminée, notamment au niveau d'encrassement prédéterminé.

12. Procédé selon la revendication 11, d'après lequel la régulation d'au moins un paramètre de fonctionnement de l'installation de traitement de contenants, notamment du dispositif de nettoyage, est effectuée sur la base du résultat de la comparaison du niveau d'encrassement résiduel déterminé avec la valeur prédéterminée.

13. Procédé selon l'une des revendications 8 - 12, comprenant, en outre, un nettoyage d'un ou de plusieurs éléments de l'installation ayant été traversés par le contenant de test (624; 825; 924; 1024).
